# EUROPÄISCHE PATENTSCHRIFT

(11) **EP 1 361 224 B1**
(45) Veröffentlichungstag und Bekanntmachung des Hinweises auf die Patenterteilung: **28.12.2005**
(21) Anmeldenummer: 03018096.2
(22) Anmeldetag: 05.06.2001
(51) Int. Cl.: C07D 403/12, C07D 401/12, A61K 31/44, A61P 35/00

(54) **Heterocyclische Hydrazone als anti-Krebs-Wirkstoffe**
Heterocyclic hydrazones for use as anti-cancer agents
Hydrazones heterocycliques comme agents anti-cancereux

(30) Priorität: 05.06.2000 AT 9772000
(43) Veröffentlichungstag der Anmeldung: 12.11.2003
(62) Teilanmeldung aus: 01942856.4
(73) Patentinhaber: Austria Wirtschaftsservice Gesellschaft mit beschränkter Haftung, 1030 Wien (AT); Hofmann, Johann, 6020 Innsbruck (AT); Heinisch, Gottfried, 6020 Innsbruck (AT); Easmon, Johnny, 6020 Innsbruck (AT); Pürstinger, Gerhard, 6020 Innsbruck (AT); Fiebig, Heinz Herbert, 79110 Freiburg (DE)
(72) Erfinder: Hofmann, Johann, Inst. f. Med. Chemie u. Biochemie, 6020 Innsbruck (AT); Heinisch, Gottfried, Institut für Pharmazie, 6020 Innsbruck (AT); Easmon, Johnny, Institut für Pharmazie, 6020 Innsbruck (AT); Pürstinger, Gerhard, Institut für Pharmazie, 6020 Innsbruck (AT); Fiebig, Heinz-Herbert, Oncotest GmbH, 79110 Freiburg (DE)
(74) Vertreter: Sonn & Partner Patentanwälte

(56) Entgegenhaltungen:
- EASMON, J. ET AL: "Thiazolyl and benzothiazolyl hydrazones derived from.alpha.-(N)- acetylpyridines and diazines: synthesis, antiproliferative activity and CoMFA studies" EUR. J. MED. CHEM. (1997), 32(5), 397-408, 1997, XP004085514
- HALL, IRIS H. ET AL: "Investigations on the mechanism of action of the novel antitumor agents 2-benzothiazolyl, 2-benzoxazolyl, and 2-benzimidazolyl hydrazones derived from 2-acetylpyridine" ARCH. PHARM. (WEINHEIM, GER.) (1999), 332(4), 115-123, 1999, XP001013551

## Beschreibung

Die vorliegende Erfindung betrifft neue 2-Benzimidazolylhydrazone, die von 2-Formylpyridin, 2-Acylpyridinen, Acetyldiazinen und Acetyl(iso)chinolinen abstammen, ein neues Verfahren zur Herstellung von 2-Benzimidazolylhydrazonen sowie deren Einsatz als nützliche Antikrebs-Therapeutika. Weiters sind diese Verbindungen auch aktiv gegen Krebszellen, die Multidrug-Resistenz zeigen.

Trotz neuen Erkenntnissen in der Tumorbiologie spielen weiterhin chirurgische Eingriffe, Bestrahlung und antitumoral wirksame Substanzen die wichtigsten Rollen in der Tumortherapie. Nachteile der bisher zur Verfügung stehenden Antitumorsubstanzen sind starke Nebenwirkungen, geringe Ansprechraten bei soliden Tumoren und die Entwicklung von Resistenzen. Insbesondere bei Colonkarzinomen, einem der häufigsten Tumore in der westlichen Welt, zeigt die Chemotherapie nur geringe Wirksamkeit. Deshalb wären wirksamere Antitumorsubstanzen wünschenswert.

Das Enzym Ribonukleotid Reduktase (RR) stellt ein wichtiges Zielmolekül in der Chemotherapie von Krebs dar. In der Absicht eine neue Klasse von RR-Inhibitoren zu entwickeln, wurde das N-N-S-Pharmacophor von α-(N)-heteroaromatischen Thiosemicarbazonen, z.B. Verbindungen (1) und (2), als Ausgangspunkt gewählt.

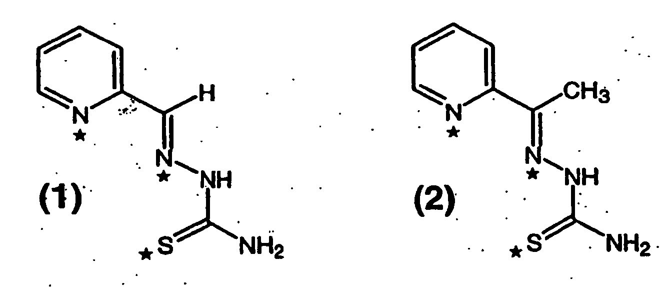

Weiters wurden 2-Benzothiazolyl- und 2-Thiazolylhydrazone, die von 2-Formylpyridin, z.B. Verbindungen (3) und (4), 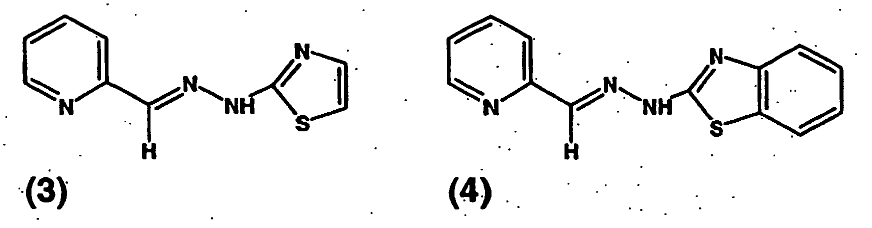 beziehungsweise 2-Acetylpyridinen, z.B. Verbindungen (5) und (6), 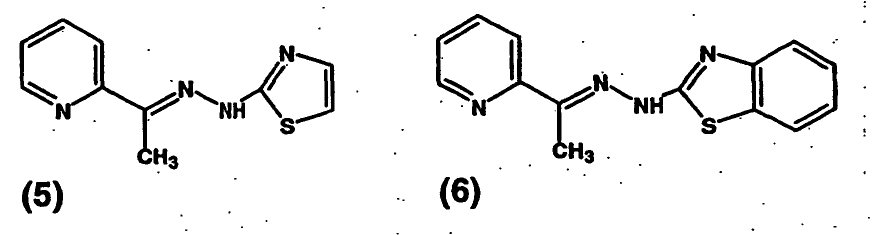 abstammen, bereits synthetisiert.

Diese Verbindungen wurden auch bereits *in vitro* gegen eine Reihe von humanen Tumor-Zelllinien getestet, wobei die bekannten Verbindungen (1) und (2) als Kontrollen dienten. Die Hydrazone (3) und (5) stellten sich dabei als 5 bis 10 mal aktiver als die bekannten Thiosemicarbazone (1) und (2) heraus (siehe *Easmon et al*., *Eur. J. Med. Chem., 32, 397 (1997)*). Weiters konnte auch bereits gezeigt werden, dass die Verbindungen (3) bis (6) keine Kreuzresistenz gegen eine Leukämie-Zelllinie aufweisen, welche die M2 Proteinuntereinheit überexprimiert. Jedoch stellte sich dabei auch heraus, dass die Verbindungen (3) bis (6) die RR nicht hemmen und dass das N-N-S Pharmakophor für diese Klasse von Wirkstoffen nicht relevant ist. Diese Vermutung wurde auch dadurch unterstützt, dass diese Verbindungen Metallionen in der N-N-N Form binden, wie die Röntgenstrukturanalyse des Nickelkomplexes der Verbindung (6) ergab.

In der Weiterführung der Bemühungen, neue Antitumor-Wirkstoffe zu entwickeln, wurden nun gemäß der vorliegenden Erfindung Hydrazone dargestellt, bei denen das 2-Benzothiazolylringsystem durch ein 2-Benzimidazolylringsystem ersetzt wurde. Das führte zu einer neuen Klasse von Hydrazonen, die potente cytotoxische und antitumorale Aktivität zeigt und auch gegen Multidrug-resistente Tumore nützlich ist.

Die antiproliferative Aktivität der neuen Substanzen wurden in verschiedenen humanen TumorZelllinien getestet. Wirksame Verbindungen wurden dann im Clonogenic Assay (Hemmung der Koloniebildung von humanen Tumortransplantaten in Softagar) untersucht.

Einige der Substanzen wurden in Mäusen getestet, denen humane CXF 280 Colon Tumorzellen direkt in die Flanken transplantiert wurden (Humane Tumor Transplantate). In allen Versuchen zeigten die Substanzen Antitumoraktivität, insbesondere gegen Colontumoren.

Bei der intensiven Suche nach Verbindungen mit Antitumoraktivität wurde nun überraschenderweise gefunden, dass neue Hydrazone, abgeleitet von (benzoannellierten) α-(N)-Formyl- und Acyl-(Di)azinen und 2-Hydrazinobenzimidazolen beachtliche Antitumoraktivität sowohl *in vitro* als auch *in vivo* zeigen.

Die vorliegende Erfindung betrifft nun neue Verbindungen der allgemeinen Formel 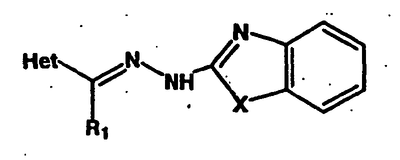 **wobei Het =** 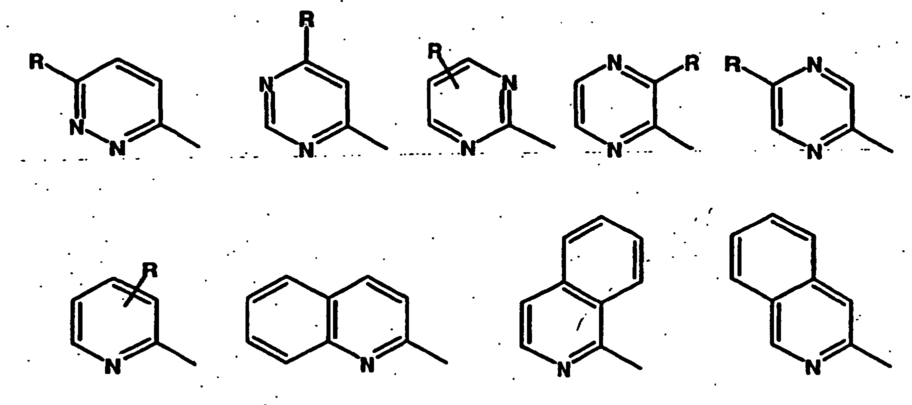 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆ H, einen aliphatischen Substituenten oder eine Phenylgruppe bedeuten,
R₁ = H, Methyl, Ethyl, Propyl, iso-Propyl, Butyl, tert.-Butyl, Cyclopropyl, Cyclohexyl, Phenyl, Benzyl, oder 2-Pyridyl, und
X = NH oder N-R₂ bedeutet, wobei R₂ = Methyl, Ethyl, Propyl, sec-Propyl, Butyl, *tert*-Butyl, Allyl, Cyclopropyl, Phenyl, Benzyl, CH₂-CH₂-O-CH₃ oder CH₂-CH₂-N(CH₃)₂ ist,
mit der Maßgabe, dass, wenn Het = , 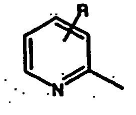 wobei R = H,
im Fall von X = NH: R₁ nicht Methyl bedeutet,
im Fall von X = N-R₂ mit R₂ = CH₃: R₁ nicht Methyl bedeutet;
sowie deren pharmazeutisch annehmbaren Salze.

Die vorliegende Erfindung betrifft ebenfalls ein Verfahren zur Herstellung einer Ver bindung der allgemeinen Formel 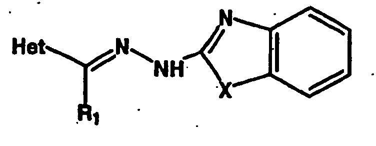 **wobei Het. =** 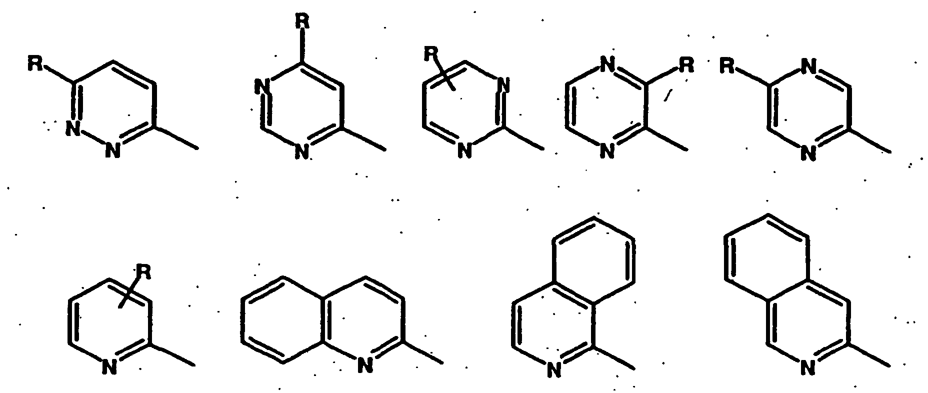 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆ H, einen aliphatischen Substituenten oder eine Phenylgruppe bedeuten,
R₁ = H, Methyl, Ethyl, Propyl, iso-Propyl, Butyl, tert.-Butyl, Cyclopropyl, Cyclohexyl, Phenyl, Benzyl, oder 2-Pyridyl, und X = NH oder N-R₂ bedeutet, wobei R₂ = Methyl, Ethyl, Propyl, sec-Propyl, Butyl, *tert*-Butyl*,* Allyl, Cyclopropyl, Phenyl, Benzyl, CH₂-CH₂-O-CH₃ oder CH₂-CH₂-N(CH₃)₂ ist, durch Umsetzen geeigneter Ketone mit passend substituierten Hydrazinen. Insbesondere können die obgenannten Verbindungen sowie geeignete Zwischenverbindungen durch das folgende Verfahren hergestellt werden: 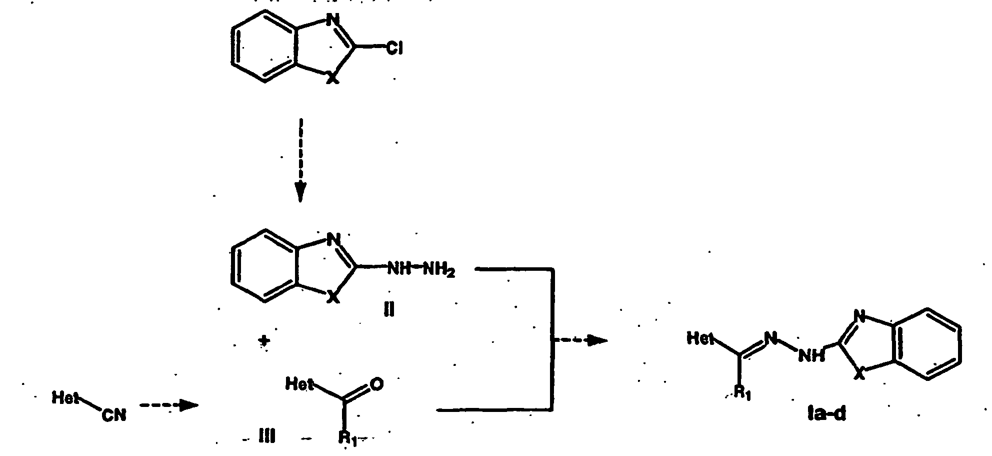 wobei Het, R, R₁, R₂, R₃, R₄, R₅, R₆ und X wie oben definiert sind.

Die Hydrazone des Typs Ia-d werden durch Erhitzen eines Ketons (III) und eines Hydrazins (II) in Methanol oder Ethanol unter Zugabe einer katalytischen Menge einer geeigneten Säure, wie z.B. Essigsäure, Salzsäure oder Schwefelsäure, dargestellt. Die Synthese kann alternativerweise auch bei Raumtemperatur durchgeführt werden, dauert dann aber mehrere Tage bis zum vollständigen Umsatz. Die Hydrazine des Typs II werden durch Erhitzen der entsprechenden 2-Chlor-Edukte mit 98%igem Hydrazinhydrat auf Rückflusstemperatur nach Standardvorschriften hergestellt, beispielsweise für X= NH siehe *Bednyagina, N. P. and Postovskii, I. Ya., Zh Obshch Khim 30, 1431, 1960; Chem Abstr 55*: *1586, (1961)* und für X = N-CH₃ siehe *Kulkarni M. V. and Patil, V. D., Arch Pharm 314, 440, (1981).*

Die Ketone des Typs III wurden durch Umsatz von entsprechenden 2-Cyano-verbindungen mit einem geeigneten Grignardreagens (RMgX), Alkyllithium- oder Phenyllithiumreagens - in Analogie zu bekannten Verfahren bzw. zur Patentliteratur (siehe *Lutz, H. et at. DE-43 06 006-A*) - synthetisiert.

### Pharmakologische Tests:

Die überraschende Antitumor-Aktivitäten der neuen bzw. bereits im Stand der Technik allgemein geoffenbarten Hydrazone der vorliegenden Erfindung werden im folgenden beschrieben. Als Kontrolle wird Hydroxyharnstoff, ein kommerziell erhältliches Krebs-Chemotherapeutikum, herangezogen.

### Hemmung desTumorzellwachstums.

Um Information über die wachstumshemmende Wirkung auf Tumorzellen zu bekommen, wurde die Hemmung des Wachstums folgender humaner Tumorzellen bestimmt: Burkitt's Lymphoma (CA 46, ATCC Nr. CRL 1648), CCRF-CEM (akute lymphoblastische Leukämie, ATCC Nr. CCL 119), K562 (chronisch myeloische Leukämie, ATCC Nr. CCL 243), HeLa (epitheloides Cervix Karzinom, ATCC Nr. CCL 2), MEXF 276L (Melanom), HT-29 (Colon Adenokarzinom, ATCC Nr. HTB 38), KB-3-1 (humanes Oral-Epidermoid Karzinom, CTCC Nr. CCL 17), KB-HU Hydroxyharnstoff resistente, Multidrug resistente KB-C1 Zellen (*Akiyama et al., Cell. Mol. Genet., 11:117-126, 1985*). Burkitt's, CCRF-CEM, HeLa und MEXF 276L Zellen wurden in RPMI 1640, HT-29 Zellen in McCoy's 5A Medium gezogen. Die KB Zelllinien wurden in Dulbecco's modified Eagle's medium (4,5 g Glucose/l) gezogen. Zu den KB-C1 Kulturen wurde jede 2. Woche 1µg Colchizin/ml, zu den Hydroxyharnstoff-resistenten KB-HU Zellen 1 mM Hydroxyharnstoff zugegeben. Die Medien wurden mit 10 % fötalem Kälberserum (ausgenommen Burkitt's Lymphom Zellen mit 15 %), 2 mM Glutamin, 50 units/ml Penicillin and 50 µg/ml Streptomycin supplementiert. Die Wachstumshemmung von HeLa, HT-29, KB und MEXF 276L Zellen wurde mit dem SRB-Assay bestimmt (*Skehan et al., J. Natl. Cancer. Inst., 82:1107-1112, 1990*). 3 000 - 10 000 Zellen wurden in 200 µl Medium pro Well in 96-Well Platten ausplattiert. Die Dosis-Wirkkurven für CCRF-CEM und Burkitt's Lymphom Zellen wurden mit dem MTT-Assay (*Mosman, J. Immunol. Methods, 65:55-63, 1983)* von Boehringer Mannheim, Mannheim, Deutschland, erstellt. Dazu wurden ca. 10 000 Zellen pro 100 µl in 96-Well Platten ausgesät. Nach einer anfänglichen Inkubation über vier Stunden wurden verschiedene Substanzkonzentrationen zugegeben und die Zellen 72 Stunden bei 37° C in wassergesättigter 95%iger Luft und 5%igem CO₂ inkubiert. Die Substanzen wurden in Dimethylsulfoxid (DMSO) gelöst. Die DMSO-Konzentration war 0,5 % und diese war für die Zellen nicht toxisch. Danach wurden die Proben fixiert, gewaschen und die Absorption in einem Microplate Reader bestimmt.

Die Ergebnisse werden in den Tabellen 1-4 wiedergegeben

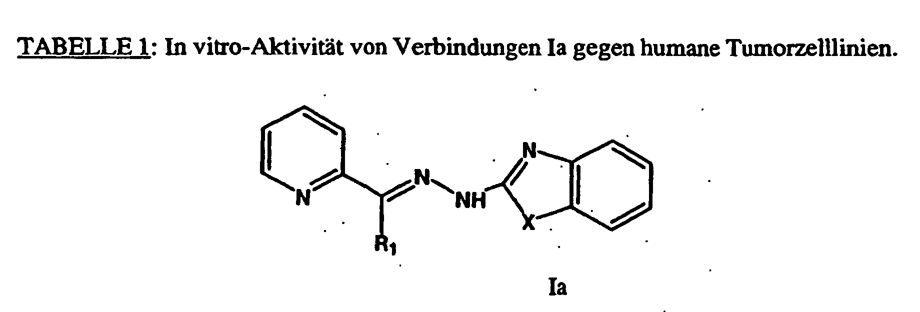

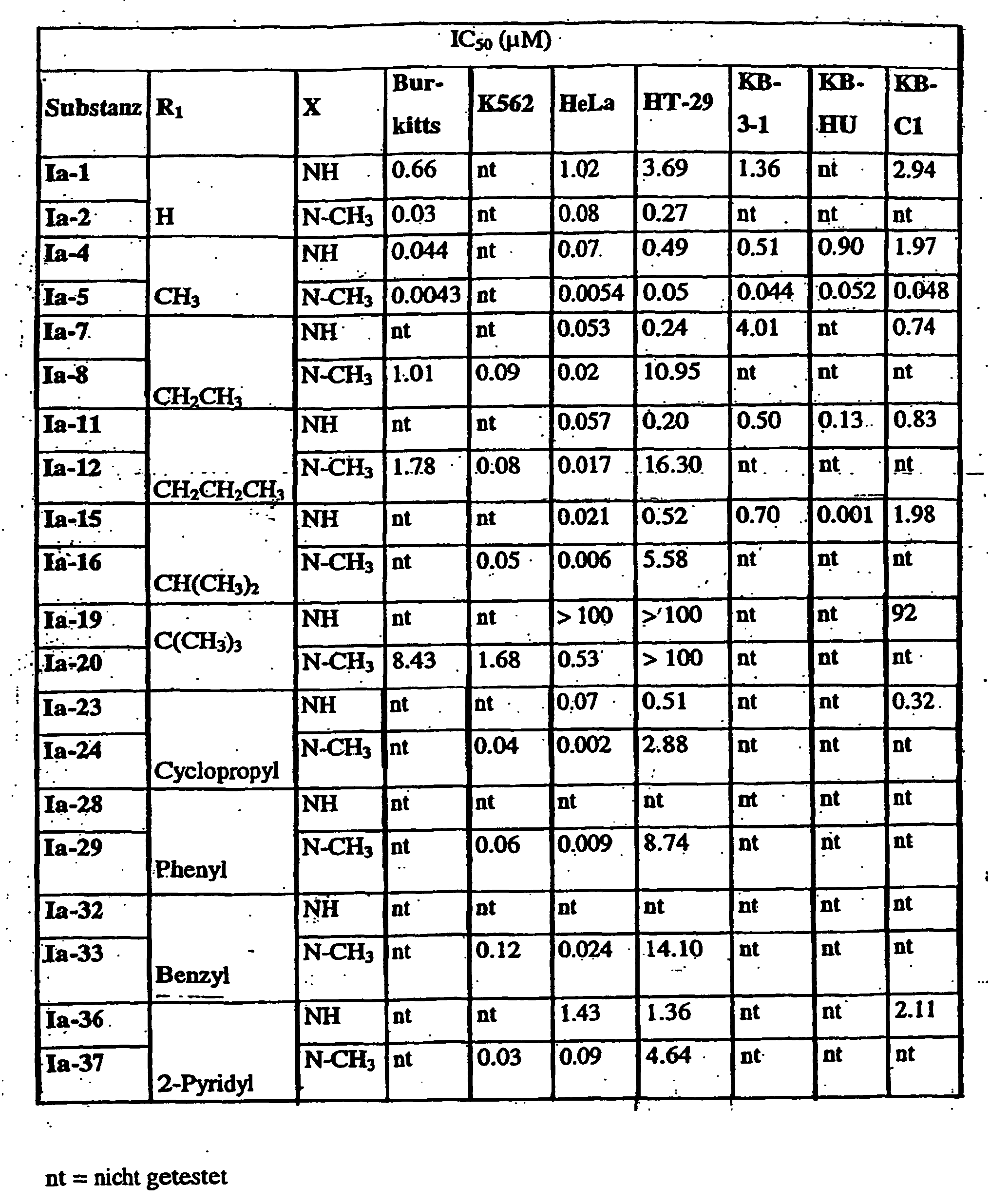

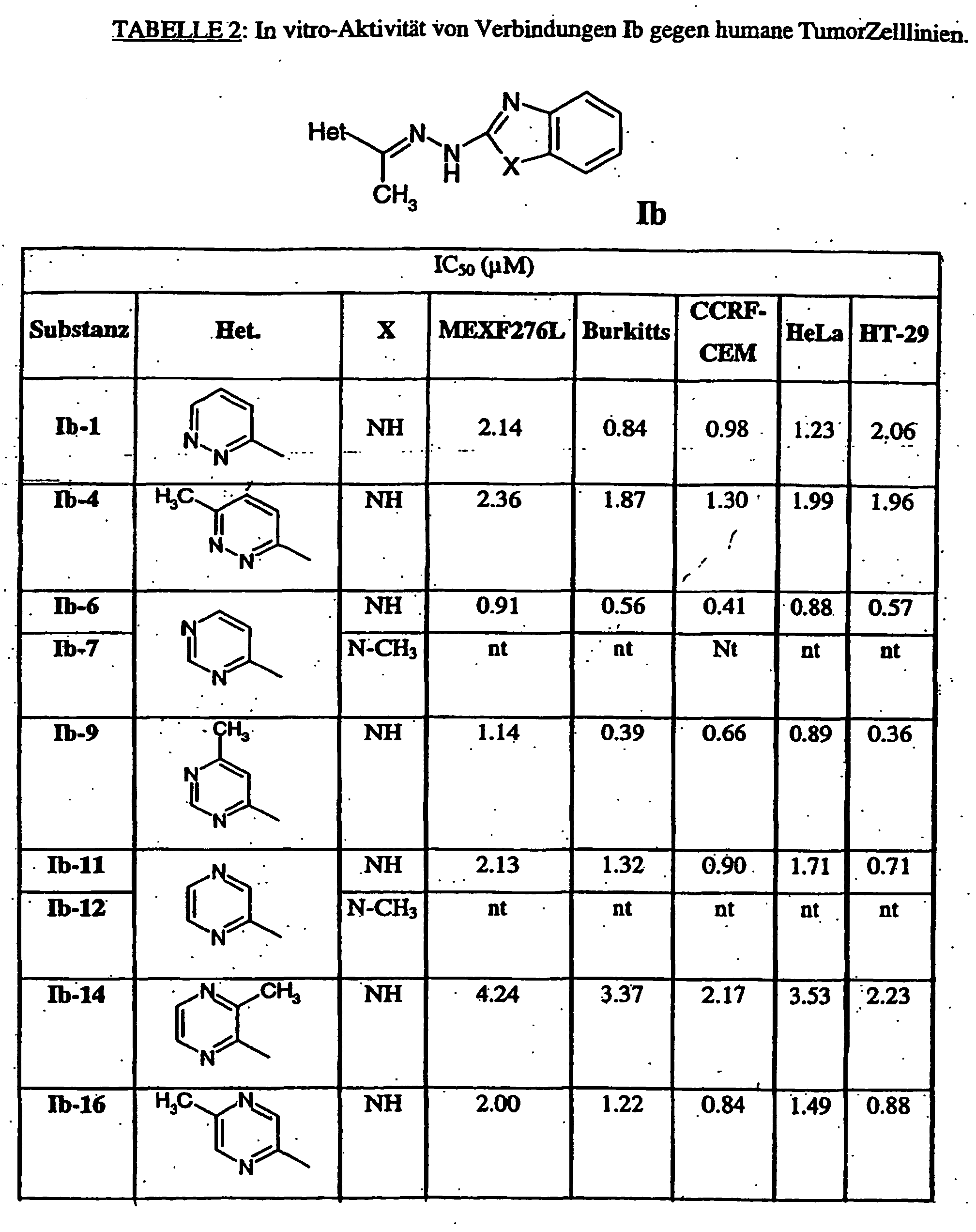

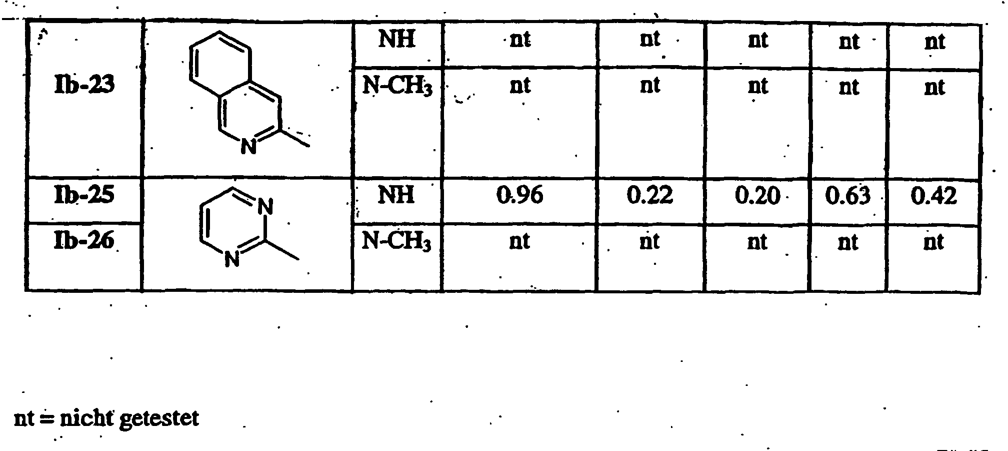

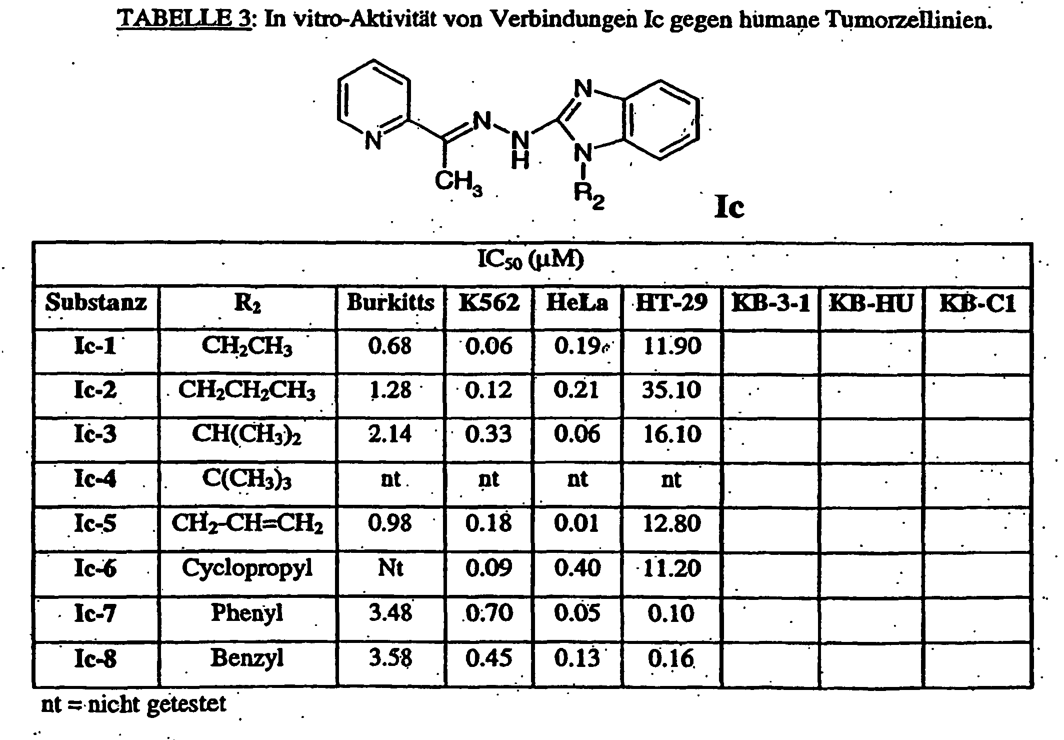

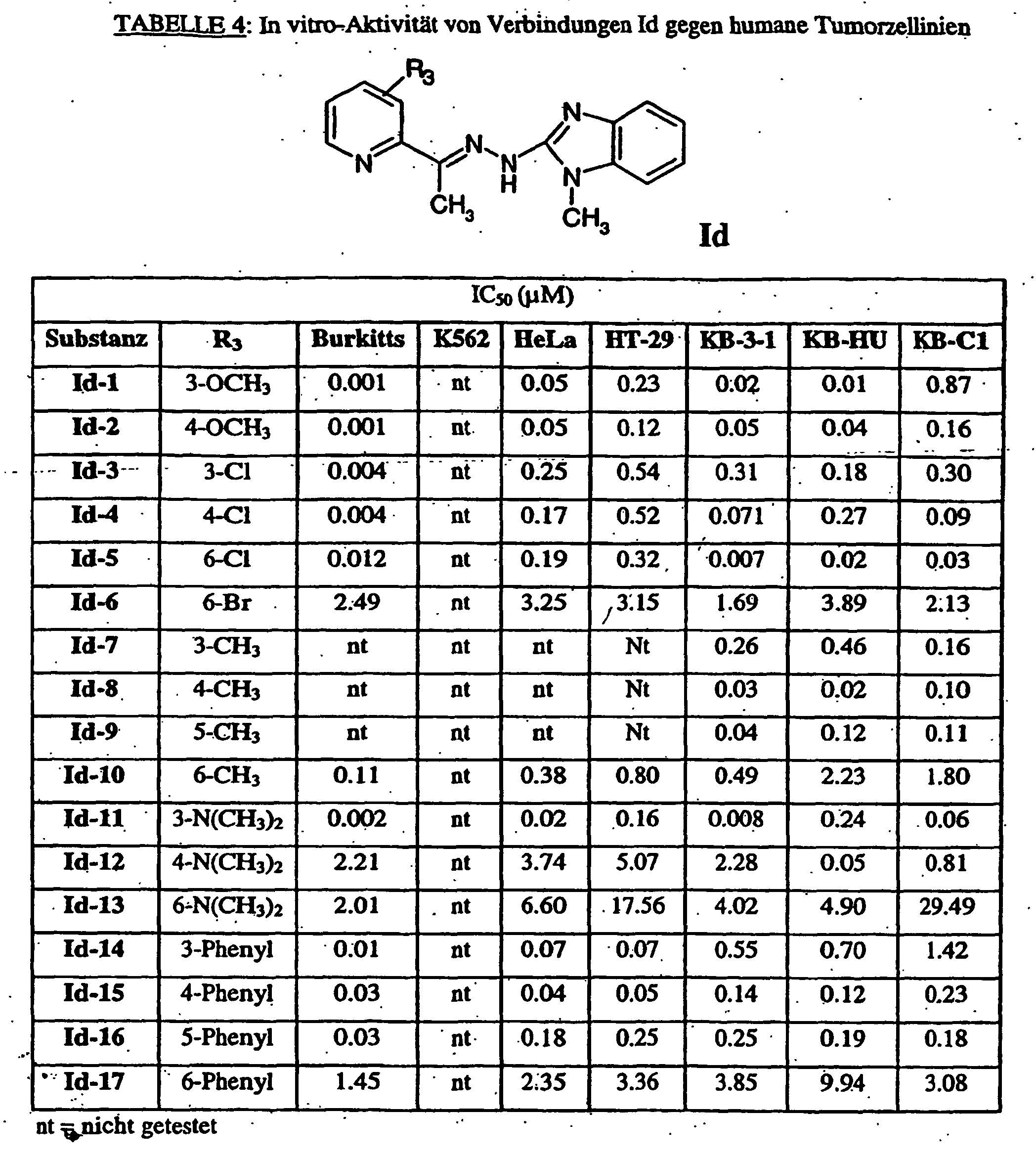

### Kolonienbildungsassay als genauere in vitro-Untersuchung

Um detaillierte Informationen darüber zu erhalten, bei welchem Tumortyp die Substanzen die beste Wachstumshemmung bewirken, wurde die Koloniebildung von humanen Tumortransplantaten getestet. Zwischen der Ansprechrate in Patienten und dem Koloniebildungsassay wurde eine ausgezeichnete Korrelation gefunden (*Scholz et al., Eur. J. Cancer* *25:901-905, 1990*). Solide humane Tumoren wurden als Transplantate in Nacktmäusen gezogen, aus diesen entfernt, mechanisch zerkleinert und danach bei 37°C für 30 Minuten in einem Enzymcocktail inkubiert, der aus Kollagenase (1,2-1,8 U/mL), DNAse (375 U/mL) und Hyaluronidase (29 U/mL) in RPMI 1640 Medium bestand. Die Zellmischung wurde durch Siebe mit 200 µm und 50 µm Porengröße passiert und danach zweimal mit PBS (phosphate buffered saline) gewaschen. Der Prozentsatz von lebenden Zellen wurde mit einer Neubauerzählkammer und Trypanblaufärbung gezählt.

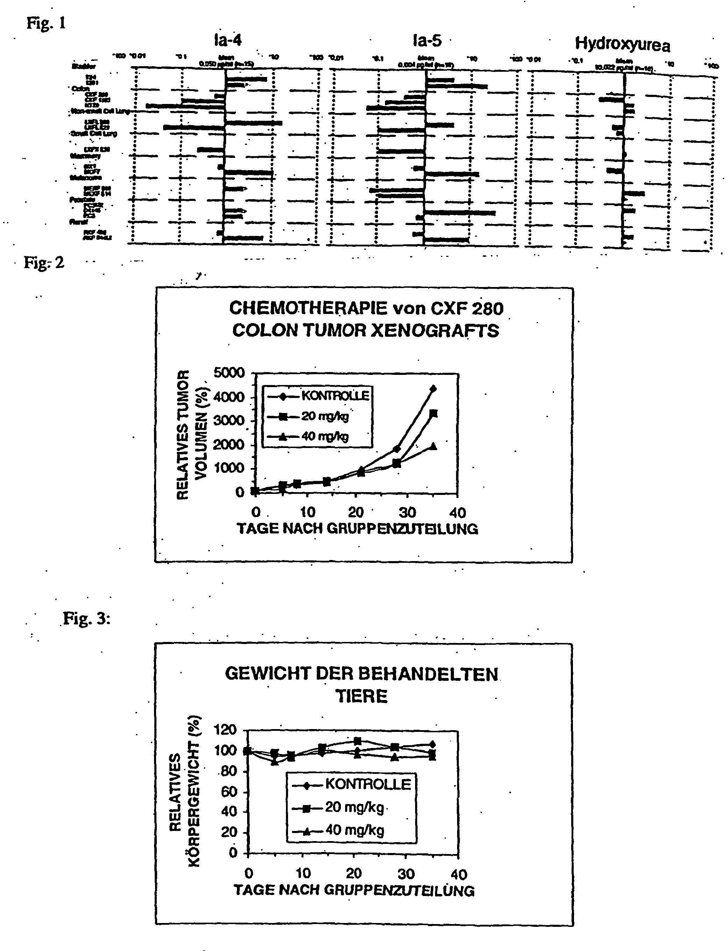

Der Koloniebildungsassay wurde mit einer von Hamburger und Salmon (*Hamburger and Salmon, Science, 197:461-463, 1977*) eingeführten und modifizierten Zweischichtagartechnik durchgeführt. Die untere Schicht bestand aus 0,2 ml Iscove's modified Dulbecco's Medium mit 20% fötalem Kälberserum und 0,75% Agar. 8x10³ bis 1,6x10⁴ Zellen wurden in dasselbe Medium und 0,4% Agar gegeben und in 24-Multiwell Platten auf die untere Schicht plattiert. Die Substanzen wurden einen Tag nach dem Ausplattieren (bei andauernder Exposition) in 0,2 ml Medium zugegeben. Jede Platte enthielt 6 Kontrollen nur mit dem Lösungsmittel und die behandelten enthielten je dreifach 6 Konzentrationen der Substanzen. Die Kulturen wurden, je nach Verdoppelungszeit der Tumorzellen, 3 bis 6 Tage bei 37 °C und 7% CO₂ in wassergesättigter Atmosphäre inkubiert. Zum Zeitpunkt der maximalen Koloniebildung mit einer Größe von 50 uM wurden diese mit einem automatischen Imageanalysesystem ausgezählt. 24 Stunden vor Auszählung wurden die lebenden Kolonien mit einer sterilen wäßrigen Lösung von 2-(4-Iodophenyl)-3-(4-nitrophenyl)-5-phenyltetrazolium chloride (1mg/ml, 100µl/Well) gefärbt. Die Ergebnisse der Tests werden in Fig. 1 bzw. in der nachfolgenden Tabelle 5 gezeigt. In Fig. 1 zeigen Säulen nach links, dass die betreffenden Zelllinien sensitiver sind als der Durchschnitt. Säulen nach rechts zeigen geringere Aktivität als der Durchschnitt. Die folgenden Zelllinien wurden untersucht:

| Tumor | Zelllinie | Histologie | Kontaktzeit mit Testverbindung (Tage) |
|---|---|---|---|
| Blase | T24 | | |
| Brust | MAXF 401NL | Adenocarcinom ER-, Pr- | 6 |
| | MCF-7 | Adenocarcinom ER+, Pr+ | 4 |
| | MDA-MB 468 | | |
| Dickdarm | HT-29 | mäßig diff. Adenocarcinom | 3 |
| | SW620 | schwach diff. Adenocarcinom | 3 |
| Dickdarm | CXF 94L | | |
| Magen | GXF 251L | Adenocarcinom | 4 |
| Lunge - small cell | DMS 114 | | |
| | DMS 273 | | |
| Lunge - non small cell | LXFA 526L | Adenocarcinom | |
| | LXFA 629L | Adenocarcinom | 4 |
| | LXFE 66L | Epidermoid Carcinom | 4 |
| | LXFL 529L | large cell Carcinom | |
| | LXFL 1072L | large cell Carcinom | |
| Melanom | MEXF 462NL | amelanoides Melanom | 4 |
| | MEXF 514NL | melanoides Melanom | 4 |
| Ovarial | OVCAR3 | Adenocarcinom | 6 |
| | OVXF 899L | | |
| Prostata | DU145 | | |
| | PC3M | | |
| Renal | RXF 486L | Hypernephrom | 4 |
| | RXF 944L | Hypemephrom | 4 |
| Gebärmutter | UXF 1138L | Carcinosarcom | 4 |
| ER = Estrogen Rezeptor , Pr = Progesteron Rezeptor, (-) = negativ, (+) = positiv | | | |

**TABELLE 5**

| (Mittelwerte aller getesteten Zellen von Fig. 1) | | | |
|---|---|---|---|
| **Substanz** | **Mittel IC**_{**50**} **(µM)** | **Mittel IC**_{**70**} **(µM)** | **Mittel IC**_{**90**} **(µM)** |
| **Ia-12** | 0.246 | 0.603 | 3.736 |
| **Ia-16** | 0.188 | 0.521 | 3.769 |
| **Ia-20** | 0.015 | 0.064 | 0.231 |
| **Ia-24** | 0.138 | 0.387 | 3.135 |
| **Ia-29** | 0.294 | 0.741 | 4.664 |
| **Ic-3** | 0.265 | 0.750 | 4.301 |
| **Ic-5** | 0.214 | 0,607 | 3.334 |
| **Ic-7** | 0.760 | 1.741 | 6.296 |
| **Id-2** | 0.080 | 0.261 | 2.851 |
| **Id-4** | 0.301 | 0.956 | 4.294 |
| **Id-5** | 3.303 | 5.740 | 9.381 |
| **Id-8** | 0.102 | 0.317 | 2.385 |
| **Id-9** | 0.199 | 0.560 | 3.793 |
| **Id-11** | 0.137 | 0.397 | 2.895 |
| **Id-15** | 0.429 | 1.017 | 5.666 |
| **Id-16** | 0.965 | 2.084 | 7.449 |

Wie aus Tabelle 5 ersichtlich zeigen die Verbindungen der vorliegenden Erfindung ausgezeichnete *in vitro* Antitumoraktivitäten (IC₅₀) gegen humane Krebszellen. Im Vergleich ist die Aktivität von Hydroxyharnstoff viel geringer als die der erfindungsgemäßen Verbindungen (vgl. Fig. 1). Das IC₇₀-Muster der Substanzen ist sehr ähnlich, woraus geschlossen werden kann, dass sie den identischen Wirkmechanismus haben. Weiters zeigen die Verbindungen **Id-2, Id-8, Ia-24, Ia-16, Ic-5, Ia-29, und Id-4** Selektivität für Dickdarm-, Brust-, Ovarial- und Gebärmutterkrebs.

Die Substanz **Ib-11** wurde auch im sog. "Hollow Fibre Assay" auf ihre Antitumoraktivität getestet. Dabei werden Mäusen bis zu 12 verschiedene Tumorzellen in durchlässigen Schläuchen implantiert und mit den erfindungsgemäßen Verbindungen behandelt. Bei diesen Versuchen wurde bestätigt, dass die Substanz **Ib-11** Antitumoraktivität zeigt.

Weiters wurde von einigen derzeit in Verwendung befindlichen Antitumorverbindungen bereits gezeigt, dass sie die Apoptose (programmierten Zelltod) von Tumorzellen induzieren können. Überraschenderweise sind auch einige der erfindungsgemäßen Verbindungen in der Lage die Apoptose zu induzieren, z.B. Verbindungen **Id-12** und **Id-17**.

In unbehandelten Burkitt's Lymphon Zellen sind durchschnittlich 1,7 % apoptotisch. Wenn diese Zellen über 48 Stunden mit den doppelten IC₅₀ Konzentrationen der Verbindung **Id-12** behandelt werden sind 60 % der Zellen apoptotisch, bei Verwendung der Verbindung **Id-17** sind es 85 %. Behandlung mit Hydroxyharnstoff als Kontrolle ergab 7,3 % Apoptose. Die Bestimmung der Apoptose wurde mit Propiumjodid durchgeführt (*Nicoletti et al., Rapid and Simple method for Measuring Thymocyte Apoptosis by Propium Iodine Staining and Flow Cytometry, J. Immunol. Methods, 139, 271-279, 1991*).

### Humane Tumor Xenografts in Nacktmäusen

Humane CXF 280 Tumorzellen wurden subcutan in die Flanken von sechs bis acht Wochen alten, weiblichen, athymischen Nacktmäusen des Balb/C Stammes, die homozygot für das Nacktgen sind, implantiert. Als die Tumoren ein Größe von ca. 5-7 mm im Durchmesser hatten, wurden die Mäuse nach dem Zufallsprinzip der Kontrollgruppe oder der zu behandelnden Gruppe zugeordnet. Die Kontrollgruppe bestand aus 4 Mäusen, die 6 auswertbare Tumore hatten. Die zu behandelnde Gruppe bestand aus 3-4 Mäusen, die 4-5 auswertbare Tumore hatten. Die Substanz **Ia-5** wurde i.p. als feine Suspension an den Tagen 0, 4 und 8 in Dosen von 60, 30 und 10 mg/kg/Tag verabreicht. Die Mäuse wurden zweimal pro Woche gewogen und das Tumorvolumen mit einem Tasterzirkel gemessen. Das Tumorvolumen wurde nach folgender Formel berechnet: Tumor Volumen (mm³) = Breite (mm)² X Länge (mm) / 2. Die Werte für das relative Tumorvolumen wurde für jeden einzelnen Tumor berechnet, indem das Tumorvolumen am Tag X (TVₓ) durch das Tumorvolumen am Tag 0 (TV₀) zum Zeitpunkt der nach dem Zufallsprinzip erfolgten Zuteilung [RTV=(TVₓ x 100)/TV₀)] dividiert wurde. Die mittleren RTV Werte wurde für die weitere Beurteilung verwendet.

Fig. 2 zeigt die Änderung des Tumorvolumens, nachdem die Substanz **Ia-5** der vorliegenden Erfindung Mäusen, denen humane Tumore transplantiert wurden, verabreicht wurde.

Fig. 3 zeigt die Änderung des Körpergewichtes im Laufe der Zeit, wenn die erfindungsgemäße Substanz **Ia-5** Mäusen, denen humane CXF 280 Colon Tumoren transplantiert wurden, verabreicht wurde (Gewichtsabnahme während der Behandlung gilt als Maß für die Toxizität der Substanz).

Die Herstellung von Verbindungen der vorliegenden Erfindung wird nun anhand der nachfolgenden Beispiele, auf die sie jedoch nicht beschränkt sein soll, erläutert. Die angegebenen NMR-Daten beziehen sich auf Messung in DMSO-*d6*.

### Beispiel 1:

### 1-(2-Pyridyl)-1-ethanon-1-(1H-benzo[d]imidazol-2-yl)hydrazon (Ia-4)

Eine Mischung von 2-Acetylpyridin (1,00 g, 8,25 mmol) und 2-Hydrazinobenzimidazol (1,22 g, 8,25 mmol) in 20 ml Methanol wird nach Zugabe von 6 Tropfen Eisessig 3 Tage bei Raumtemperatur gerührt. Die Reaktionskontrolle erfolgt mittels Dünnschichtchromatographie (Polygram Sil G/UV₂₅₄ Fertigfolien; Laufmittel: CH₂Cl₂:MeOH (12:1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit 50%igem Methanol gewaschen und getrocknet. Das Produkt wird aus einem Gemisch aus Ethylacetat und Diisopropylether umkristallisiert. Ausbeute: 1,45 g (70 % d.Th.).

| C₁₄H₁₃N₅ (251.29) | | | | |
|---|---|---|---|---|
| CHN | Ber. | C 66,92% | H 5,21% | N 27,87% |
| | Gef. | C 66,79% | H 5,47% | N 27,64% |

¹H-NMR (δ, ppm) = 2,39 (s, 3H, CH₃), 6,94-7,04 (m, 2H, arom. H), 7,20-7,36 (m, 3H, arom. H), 7,82 (ddd, 1H, Pyridin-H4, J=8,1, 7,4, 1,9 Hz), 8,48 (br d, 1H, arom. H), 8,56 (ddd, 1H, Pyridin-H6, J=4,9, 1,8, 0,8 Hz), 10,85 (br s, 1H, NH), 11,51 (br s, 1H, NH).

### Beispiel 2:

### E/Z-Cyclopropyl(2-pyridyl)methanon-(1-methyl-1H-benzo[d]imidazol-2-yl)hydrazon (Ia-24)

Eine Mischung von Cyclopropyl(2-pyridyl)methanon (1,00 g, 7,40 mmol) und 1-Methyl-2-hydrazinobenzimidazol (1,20 g, 7,40 mmol) in 20 ml Methanol wird nach Zugabe von 6 Tropfen Eisessig 3 Tage bei Raumtemperatur gerührt. Die Kontrolle erfolgt mittels Dünnschichtchromatographie (Polygram Sil G/UV₂₅₄ Fertigfolien; Laufmittel: CH₂Cl₂:MeOH (12:1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit 50%igem Methanol gewaschen und getrocknet. Das Produkt wird aus einem Gemisch aus Methanol und Wasser umkristallisiert. Ausbeute: 0,97 g (45 % d.Th.).

| C₁₇H₁₇N₅ (291.36) | | | | |
|---|---|---|---|---|
| CHN | Ber. | C 70,07% | H 5,88% | N 24,05% |
| | Gef. | C 70,37% | H 6,08% | N 24,04% |

¹H-NMR (δ ppm) = 0,71-1,04 (m, 4H, Cyclopropyl-CH₂-CH₂), 2,08-2,35 (m, 1H, Cyclopropyl-CH), 2,95-3,05 (m, 1H. Cyclopropyl-CH), 3,25 (s, 1H, N-CH₃), 3,42 (s, 1H, N-CH₃), 3,81 (s, 1H, N-CH₃), 6,91-7,17 (m, Pyridin-H + arom. H), 7,21-7,40 (m, Pyridin-H + arom. H), 7,54-7,62 (m, Pyridin-H + arom. H) 7,70-7,84 (m, Pyridin-H + arom. H), 8,11-8,28 (m, Pyridin-H + arom. H), 8,45 (br. d, 1H, Pyridin-H6), 8,64 (br. d, 1H, Pyridin-H6), 8,85 (br. d, 1H, Pyridin-H6), 10,64 (br. s, 1H, NH), 10,88 (br. s, 1H, NH), 14,61 (br. s, 1H, NH).

### Beispiel 3:

### 1-(4-Pyrimidinyl)-1-ethanon-1-(1H-benzo[d]imidazol-2-yl)hydrazon (Ib-6)

Eine Mischung von 4-Acetylpyrimidin (0,412 g, 3,37 mmol) und 2-Hydrazinobenzimidazol (0,50 g, 3,37 mmol) in 15 ml Methanol wird nach Zugabe von 5 Tropfen Eisessig unter Rückfluss erhitzt, bis die Reaktionskontrolle mittels Dünnschichtchromatographie (Polygram Sil G/UV₂₅₄ Fertigfolien; Laufmittel: CH₂Cl₂:MeOH (10:1)) keinen weiteren Umsatz anzeigte. Das Reaktionsgemisch wird über Nacht im Kühlschrank bei ca. 5 °C aufbewahrt. Der Niederschlag wird abfiltriert und aus einer Mischung aus Ethylacetat und Diisopropylether umkristallisiert. Ausbeute: 0,65 g (76 % d.Th.).

| C₁₃H₁₂N₆ (252.28) | | | | |
|---|---|---|---|---|
| CHN | Ber. | C 61,89% | H4,79% | N33,31% |
| | Gef. | C 61,79% | H 4,82% | N 33,24% |

¹H-NMR (δ, ppm) = 2,36 (s, 3H), 7,01-7,07 (m, 2H), 7,20-7,27 (m, 2H), 8,50 (dd, 1H), 8,75 (d, 1H), 9,14 (d, 1H), 10,67 (br. s, 2H).

### Beispiel 4:

### 1-(3-Isochinolinyl)-1-ethanon-1-(1-Methyl-1H-benzo[d]imidazol-2-yl)hydrazon (Ib-23)

Eine Mischung von 3-Acetylisochinolin (1,01 g, 5,9 mmol) und 1-Methyl-2-hydrazinobenzimidazol (0,96 g, 5,55 mmol) in 15 ml Methanol wird nach Zugabe von 5 Tropfen Eisessig ca. 7 Tage bei Raumtemperatur gerührt. Die Reaktionskontrolle erfolgt mittels Dünnschichtchromatographie (Polygram Sil G/UV₂₅₄ Fertigfolien; Laufmittel: Petrolether:Ethylacetat (3:7)). Das Reaktionsgemisch wird im Tiefkühlschrank bei ca. -20 °C über Nacht aufbewahrt. Der Niederschlag wird abfiltriert, mit Ether gewaschen und getrocknet. Das Produkt wird aus einem Gemisch aus Ethylacetat und Petrolether umkristallisiert. Ausbeute: 1,66 g (95 % d.Th.).

| C₁₉H₁₇N₅ (315.38) | | | | |
|---|---|---|---|---|
| CHN | Ber. | C 72,36% | H 5,43% | N 22,21% |
| | Gef. | C 72,28% | H 5,31% | N 22,45% |

¹H-NMR (δ, ppm) = 2,54 (s, 3H), 3,32 (s, 3H), 3,48 (s, 3H), 6,99-7,06 (m, 2H), 7,11-7,18 (m, 2H), 7,62 (ddd, 1H), 7,77 (ddd, 1H), 7,97 (d, 1H), 8,10 (d, 1H), 8,79 (s, 1H), 9,31 (s, 1H), 11,06 (s, 1H).

### Beispiel 5:

### 1-(2-pyridyl)-1-ethanon-1-(1-ethyl-1H-benzo[d]imidazol-2-yl)hydrazon (Ic-1)

Eine Mischung von 2-Acetylpyridin (0,62 g, 5,11 mmol) und 1-Ethyl-2-hydrazinobenzimidazol (0,90 g, 5,11 mmol) in 20 ml Methanol wird nach Zugabe von 6 Tropfen Eisessig 24 Stunden bei Raumtemperatur gerührt. Die Reaktionskontrolle erfolgt mittels Dünnschichtchromatographie (Polygram Sil G/UV₂₅₄ Fertigfolien; Laufmittel: CH₂Cl₂:MeOH (12:1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit 50%igem Methanol gewaschen und getrocknet. Das Produkt wird aus einem Gemisch aus Methanol und Wasser umkristallisiert. Ausbeute: 0,99 g (70 % d.Th.).

| C₁₆H₁₇N₅ (279.35) | | | | |
|---|---|---|---|---|
| CHN | Ber. | C 68,80% | H 6,13% | N 25,07% |
| | Gef. | C 68,79% | H 6,23% | N 25,24% |

¹H-NMR (δ, ppm) = 1,29 (t, 3H), 2,43 (s, 3H), 4,03 (q, 2H). 6,98-7,20 (m, 4H). 7,31 (ddd, 1H), 7,76 (ddd, 1H), 8,48 (d, 1H), 8,53 (ddd, 1H), 11,06 (br. s, 1H).

### Beispiel 6:

### 1-(2-Pyridyl)-1-ethanon-1-(1-phenyl-1H-benzo[d]imidazol-2-yl)hydrazon (Ic-7)

Eine Mischung von 2-Acetylpyridin (1,00 g, 8,26 mmol) und 1-Phenyl-2-hydrazinobenzmidazol (1,85 g, 8,26 mmol) in 20 ml Methanol wird nach Zugabe von 6 Tropfen Eisessig 2 Tage bei Raumtemperatur gerührt. Die Reaktionskontrolle erfolgt mittels Dünnschichtchromatographie (Polygram Sil G/UV₂₅₄ Fertigfolien; Laufmittel: CH₂Cl₂:MeOH (12:1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit 50%igem Methanol gewaschen und getrocknet. Das Produkt wird aus einem Gemisch aus Methanol und Wasser umkristallisiert. Ausbeute: 1,35 g (50 *%* d.Th.).

| C₂₀H₁₇N₅ (327.39) | | | | |
|---|---|---|---|---|
| CHN | Ber. | C 73,37% | H 5,23% | N 21,39% |
| | Gef. | C 73,56% | H 5,39% | N 21,56% |

¹H-NMR (δ, ppm) = 2,29 (s, 3H), 6,92-7,84 (m, 11H), 8,46-8,58 (m, 2H), 11,25 (br. s, 1H).

### Beispiel 7:

### 1-(3-Methoxy-2-pyridyl)-1-ethanon-1-(1-methyl-1H-benzo[d]imidazol-2-yl)hydrazon (Id-1)

Eine Mischung von 2-Acetyl-3-methoxypyridin (0,50 g, 3,31 mmol) und 1-Methyl-2-hydrazinobenzimidazol (0,54 g, 3,31 mmol) in 10 ml Methanol wird nach Zugabe von 6 Tropfen Eisessig 3 Tage bei Raumtemperatur gerührt. Die Reaktionskontrolle erfolgt mittels Dünnschichtchromatographie (Polygram Sil G/UV₂₅₄ Fertigfolien; Laufmittel: CH₂Cl₂:MeOH (12: 1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit Wasser gewaschen und getrocknet. Das Produkt wird aus einem Gemisch aus 20 ml Methanol und 10 ml Wasser umkristallisiert. Ausbeute: 0,87 g (89 % d.Th.).

| C₁₆H₁₇N₅ (295.34) | | | | |
|---|---|---|---|---|
| CHN | Ber. | C 65,07% | H 5,80% | N 23,71% |
| | Gef. | C 63,60% | H 5,64% | N 23,21% |
| | x 0,36 H₂O | C 63,67% | H 5,92% | N 23,20% |

¹H-NMR (δ, ppm) = 2,29 (br. s, 3H, *E*-isomer), 2,57 (br. s, 3H, *Z*-isomer), 3,49 (br. s, 3H, *Z*-isomer), 4,42 (br. s, 3H, *E*-isomer), 3,81 (br. s, 3H), 6,84-7,10 (m, 4H),7,31 (dd, 1H), 7,46 (dd, 1H), 8,16 (dd, 1H), 10,59 (br. s, 1H, *E*-isomer), 13,25 (br. s, 1H, *Z*-isomer).

### Beispiel 8:

### 1-(4-Chlor-2-pyridyl)-1-ethanon-1-(1-methyl-1H-benzo[d]imidazol-2-yl)hydrazon (Id-4)

Eine Mischung von 2-Acetyl-4-chlorpyridin (0,50 g, 3,20 mmol) und 1-Methyl-2-hydrazinoenzimidazol (0,52 g, 3,20 mmol) in 5 ml Methanol wird nach Zugabe von 6 Tropfen Eisessig 4 Tage bei Raumtemperatur gerührt. Die Reaktionskontrolle erfolgt mittels Dünnschichtchromatographie (Polygram Sil G/UV₂₅₄ Fertigfolien; Laufmittel: CH₂Cl₂:MeOH (12:1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit Wasser gewaschen und getrocknet. Das Produkt wird aus einem Gemisch aus Methanol und Wasser umkristallisiert. Ausbeute: 0,60 g (62 % d.Th.).

| C₁₅H₁₄ClN₅ (327.39) | | | | |
|---|---|---|---|---|
| CHN | Ber. | C 60,10% | H 4,71% | N 23,36% |
| | Gef. | C 58,40% | H 4,55% | N 22,76% |
| | x 0,47 H₂O | C 58,45% | H 4,89% | N 22,72% |

¹H-NMR (δ, ppm) = 2,40 (s, 3H), 3,49 (s, 3H), 6,99-7,22 (m, 4H), 7,37 (dd, 1H), 8,50 (d, 1H), 8,56 (d, 1H), 11,25 (br. s, 1H).

### Beispiel 9:

### 1-(5-Methyl-2-pyridyl)-1-ethanon-1-(1-methyl-1H-benzo[d]imidazol-2-yl)hydrazon (Id-9)

Eine Mischung von 2-Acetyl-5-methylpyridin (1,00 g, 7,40 mmol) und 1-Methyl-2-hydrazinobenzimidazol (1,20 g, 7,40 mmol) in 20 ml Methanol wird nach Zugabe von 6 Tropfen Eisessig 3 Tage bei Raumtemperatur gerührt. Die Reaktionskontrolle erfolgt mittels Dünnschichtchromatographie (Polygram Sil G/UV₂₅₄ Fertigfolien; Laufmittel: CH₂Cl₂:MeOH (12:1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit 50%igem Methanol gewaschen und getrocknet. Das Produkt wird aus einem Gemisch aus Methanol und Wasser umkristallisiert. Ausbeute: 1,54 g (73 % d.Th.).

| C₁₆H₁₇N₅ (279.34) | | | | |
|---|---|---|---|---|
| CHN | Ber. | C 68,80% | H 6,13% | N 25,07% |
| | Gef. | C 68,58% | H 6,42% | N 24,97% |

¹H-NMR (δ, ppm) = 2,23 (s, 3H), 2,40 (s, 3H), 3,46 (s, 3H), 6,93-7,14 (m, 4H), 7,59 (dd, 1H), 8,34-8,42 (m, 2H), 11,00 (br. s, 1H).

### Beispiel 10:

### 1-(6-Phenyl-2-pyridyl)-1-ethanon-1-(1-methyl-1H-benzo[d]imidazol-2-yl)hydrazon (Id-17)

Eine Mischung von 2-Acetyl-6-phenylpyridin (0,50 g, 2,53 mmol) und 1-Methyl-2-hydrazinobenzimidazol (0,41 g, 2,53 mmol) in 10 ml Methanol wird nach Zugabe von 6 Tropfen Eisessig 12 Stunden bei Raumtemperatur gerührt. Die Reaktionskontrolle erfolgt mittels Dünnschichtchromatographie (Polygram Sil G/UV₂₅₄ Fertigfolien; Laufmittel: CH₂Cl₂:MeOH (12:1)). Das Reaktionsgemisch wird dann mit destilliertem Wasser verdünnt bis sich ein Niederschlag bildet und 24 Stunden im Kühlschrank bei ca. 5 °C aufbewahrt. Der Niederschlag wird abfiltriert, mehrere Male mit Wasser gewaschen und getrocknet. Das Produkt wird aus einem Gemisch aus Methanol und Wasser umkristallisiert. Ausbeute: 0,51 g (59 % d.Th.).

| C₂₁H₁₉N₅ (341.42) | | | | |
|---|---|---|---|---|
| CHN | Ber. | C 73,88% | H 5,61% | N 20,51% |
| | Gef. | C 70,24% | H 5,93% | N 19,44% |
| | x 0,92 H₂O | C 70,25% | H 5,91% | N 19,44% |

¹H-NMR (δ, ppm) = 2,55 (s, 3H), 3,49 (br. s, 3H), 6,95-7,20 (m, 4H), 7,38-7,58 (m, 3H), 7,80-7,85 (m, 2H), 8,15-8,20 (m, 2H), 8,44-8,50 (m, 1H),11,10 (br. s, 1H).

## Patentansprüche

1. Verbindung der allgemeinen Formel: 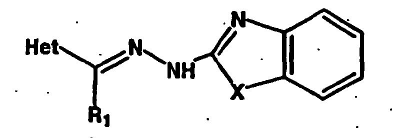 wobei Het. = 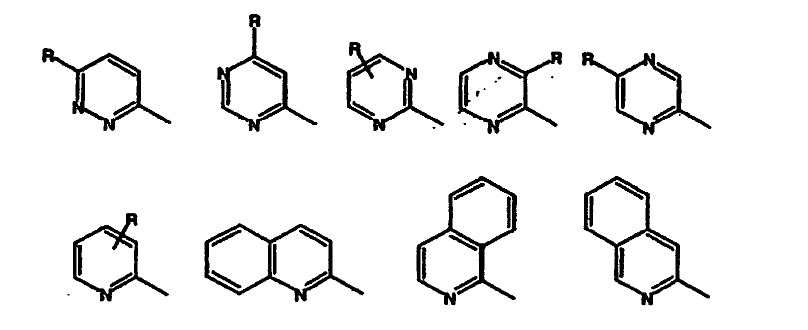 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆H, einen aliphatischen Substituenten oder eine Phenylgruppe bedeuten,
R₁ = H, Methyl, Ethyl, Propyl, *iso*-Propyl, Butyl, *tert.*-Butyl, Cyclopropyl, Cyclohexyl, Phenyl, Benzyl, oder 2-Pyridyl, und
X = NH oder N-R₂ bedeutet, wobei R₂ = Methyl, Ethyl, Propyl, *sec-Propyl,* Butyl, *tert*-Butyl, Allyl, Cyclopropyl, Phenyl, Benzyl, CH₂-CH₂-O-CH₃ oder CH₂-CH₂-N(CH₃)₂ ist,
mit der Maßgabe, dass, wenn Het = , 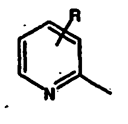 wobei R = H,
im Fall von X = NH: R₁ nicht Methyl bedeutet,
im Fall von X = N-R₂ mit R₂ = CH₃: R₁ nicht Methyl bedeutet;
sowie deren pharmazeutisch annehmbaren Salze.

2. Verbindung nach Anspruch 1, nämlich 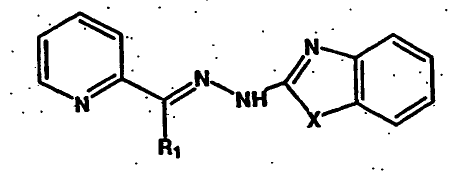 wobei R₁ = H, Methyl, Ethyl, Propyl, *iso*-Propyl, Butyl, *tert.*-Butyl*,* Cyclopropyl, Cyclohexyl, Phenyl, Benzyl oder 2-Pyridyl und X = NH oder NCH₃,
mit der Maßgabe, dass,
wenn X = NH: R₁ nicht Methyl bedeutet, und
wenn X = NCH₃: R₁ nicht Methyl bedeutet;
sowie deren pharmazeutisch annehmbaren Salze.

3. Verbindung nach Anspruch 1, nämlich 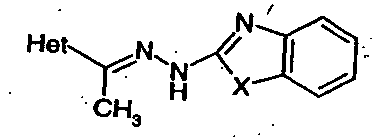 **wobei Het =** 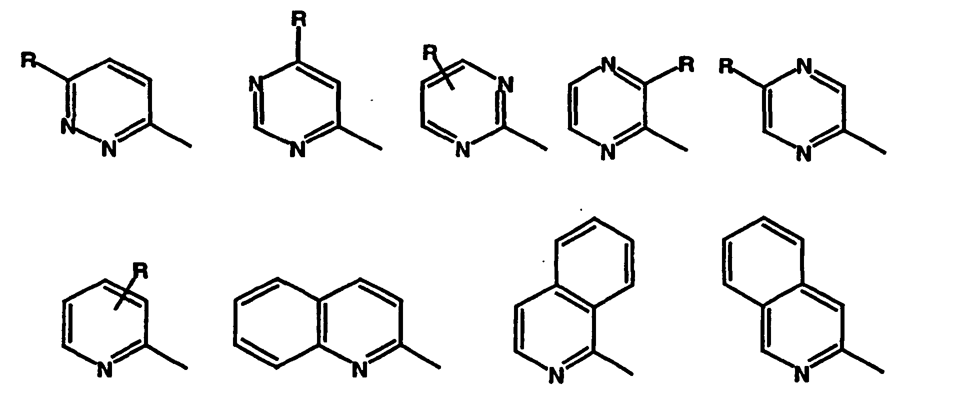 R = H oder CH₃, X = NH oder N-CH₃,
mit der Maßgabe, dass, wenn Het = 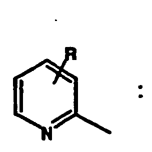 R nicht H bedeutet,
sowie deren pharmazeutisch annehmbaren Salze.

4. Verbindung nach Anspruch 1, nämlich 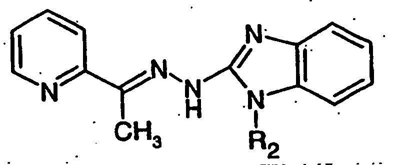 wobei R₂ = Ethyl, Propyl, sec-Propyl, Butyl, *tert-Butyl,* Allyl, Cyclopropyl, Phenyl, Benzyl oder CH₂-CH₂-O-CH₃, CH₂-CH₂-N(CH₃)₂ bedeutet, sowie deren pharmazeutisch annehmbaren Salze.

5. Verbindung nach Anspruch 1, nämlich 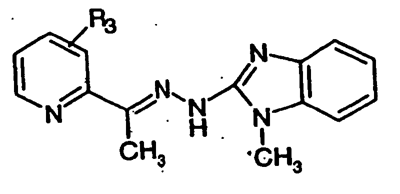 wobei R₃ einen 3, 4, 5, oder 6-Substituent darstellt und die Bedeutung OCH₃, OH, Cl, Br, F, CH₃, CF₃, NO₂, NH₂, NHCOCH₃, N(CH₃)₂, Phenyl, CN, C=NH(NH₂), C=S(NH₂), C=NH(NHOH), COOH, oder COOR₄ hat, wobei R₄ einen aliphatischen Rest, eine Phenylgruppe oder CONR₅R₆ darstellt, wobei R₅ und R₆H, einen aliphatischen Substituenten oder eine Phenylgruppe bedeuten, sowie deren pharmazeutisch annehmbaren Salze.

6. Verfahren zur Herstellung einer Verbindung der allgemeinen Formel 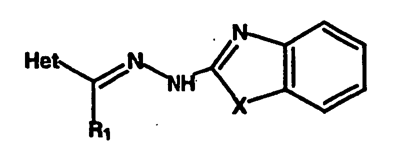 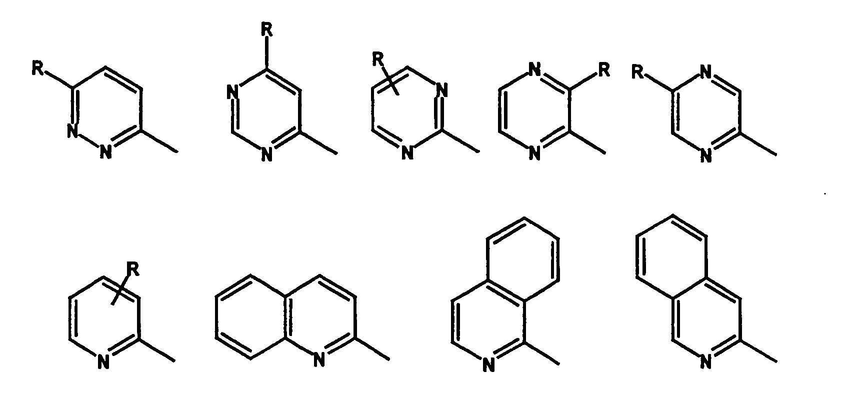 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆H, einen aliphatischen Substituenten oder eine Phenylgruppe bedeuten,
R₁ = H, Methyl, Ethyl, Propyl, iso-Propyl, Butyl, *tert*.-Butyl, Cyclopropyl, Cyclohexyl, Phenyl, Benzyl, oder 2-Pyridyl, und X = NH oder N-R₂ bedeutet, wobei R₂ = Methyl, Ethyl, Propyl, *sec*-Propyl, Butyl, *tert*-Butyl, Allyl, Cyclopropyl, Phenyl, Benzyl, CH₂-CH₂-O-CH₃ oder CH₂-CH₂-N(CH₃)₂ ist, **dadurch gekennzeichnet, dass** ein Keton der allgemeinen Formel (III) 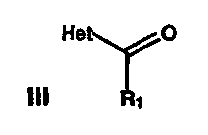 worin Het und R₁ die oben angegebene Bedeutung haben, mit einem Hydrazin der allgemeinen Formel (II) 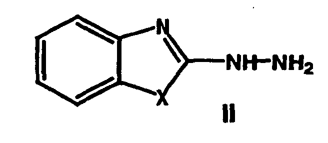 worin X die oben angegebene Bedeutung hat, umgesetzt wird.

7. Verfahren nach Anspruch 6, **dadurch gekennzeichnet, dass** die Umsetzung in Methanol oder Ethanol durchgeführt wird.

8. Verfahren nach Anspruch 6 oder 7, **dadurch gekennzeichnet, dass** die Umsetzung in Gegenwart einer katalytischen Menge einer geeigneten Säure, wie Essigsäure, Salzsäure oder Schwefelsäure, durchgeführt wird.

9. Verbindung der allgemeinen Formel: 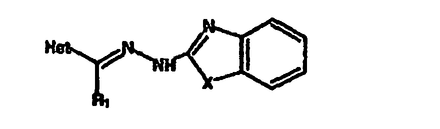 wobei Het.= 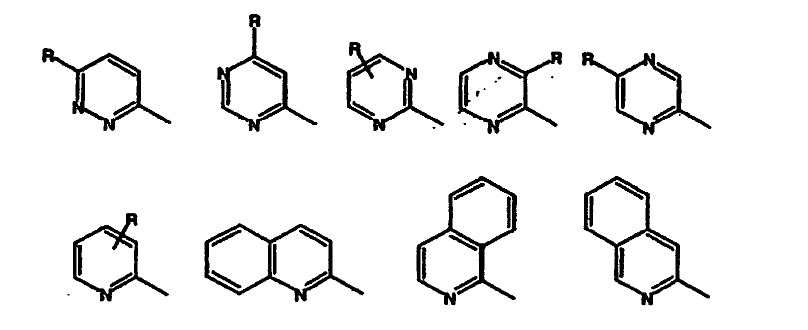 und wobei R = H, CH₃, OCH₃, OH, CL, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆H, einen aliphatischen Substituenten oder eine Phenylgruppe bedeuten.
R₁ = H, Methyl, Ethyl, Propyl, iso-Propyl, Butyl, ter.-Butyl, Cyclopropyl, Cyclohexyl, Phenyl, Benzyl, oder 2-Pyridyl, und X = NH oder N-R₂ bedeutet, wobei R₂ = Methyl, Ethyl, Propyl, sec-Propyl, Butyl, *tert*-Butyl*,* Allyl, Cyclopropyl, Phenyl, Benzyl, CH₂-CH₂-O-CH₃ oder CH₂-CH₂-N(CH₃)₂ ist, sowie deren pharmazeutisch annehmbaren Salze zur Verwendung als Arzneimittel
mit der Maßgabe, dass, wenn Het = , 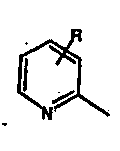 wobei R = H,
im Fall von X = NH: R₁ nicht Methyl bedeutet,
im Fall von X = N-R₂ mit R₂ = CH₃: R₁ nicht Methyl bedeutet;
sowie deren pharmazeutisch annehmbaren Salze.

10. Verbindung wie in Anspruch 9 definiert, zur Verwendung als Antikrebs-Therapeutikum.

11. Verbindung wie in Anspruch 9 definiert, zur Verwendung als Antitumor-Therapeutikum.

12. Verwendung einer Verbindung wie in Anspruch 9 definiert bei der Herstellung eines Medikaments zur Behandlung von Dickdarm-, Brust- Ovarial- oder Gebärmutterkrebs.

## Claims

1. A compound of the general formula 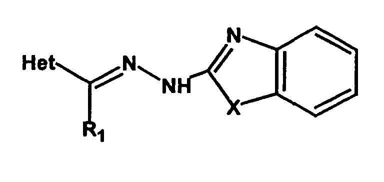 wherein Het = 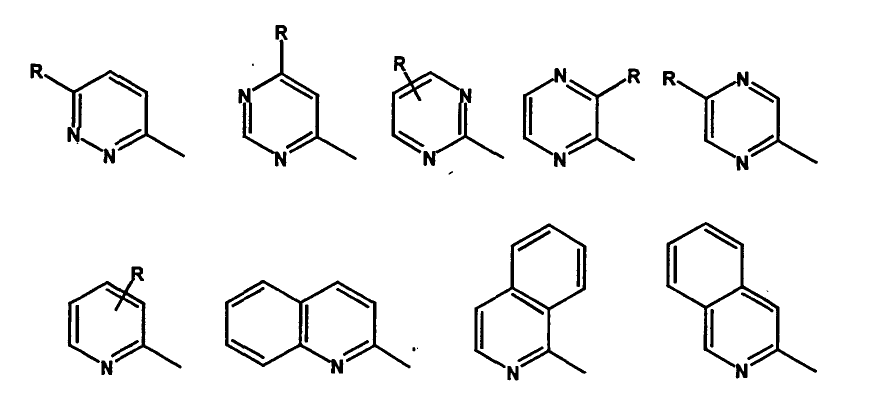 and wherein R = H, CH₃, OCH₃, OH, Cl, Br, F, CF₃, NO₂, NH₂, NHCOCH₃, N(CH₃)₂, phenyl, CN, C=NH(NH₂), C=S(NH₂), C=NH(NHOH), COOH or COOR₄, wherein R₄ = an aliphatic residue or a phenyl group, or CONR₅R₆, wherein R₅ and R₆ are H, an aliphatic substituent or a phenyl group,
R₁ = H, methyl, ethyl, propyl, *iso*-propyl, butyl, *tert.*-butyl, cyclopropyl, cyclohexyl, phenyl, benzyl or 2-pyridyl, and
X = NH or N-R₂, wherein R₂ = methyl, ethyl, propyl, sec.-propyl, butyl, tert.-butyl, allyl, cyclopropyl, phenyl, benzyl, CH₂-CH₂-O-CH₃ or CH₂-CH₂-N(CH₃)₂,
with the proviso that if Het= 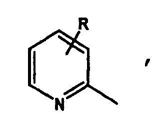 wherein R = H,
in case X = NH: R₁ is not methyl,
in case X = N-R₂ with R₂ = CH₃: R₁ is not methyl;
as well as the pharmaceutically acceptable salts thereof.

2. A compound according to claim 1, namely 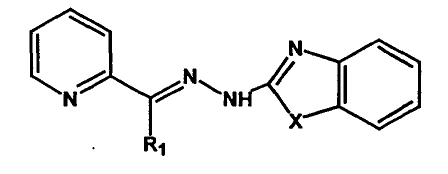 wherein R₁ = H, methyl, ethyl, propyl, *iso*-propyl, butyl, *tert*.-butyl, cyclopropyl, cyclohexyl, phenyl, benzyl or 2-pyridyl, and X = NH or NCH₃,
with the proviso that
in case X = NH: R₁ is not methyl, and
in case X = NCH₃: R₁ is not methyl;
as well as the pharmaceutically acceptable salts thereof.

3. A compound according to claim 1, namely 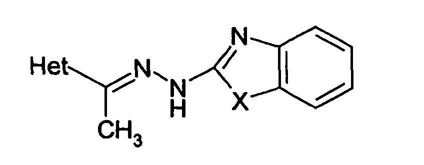 wherein Het = 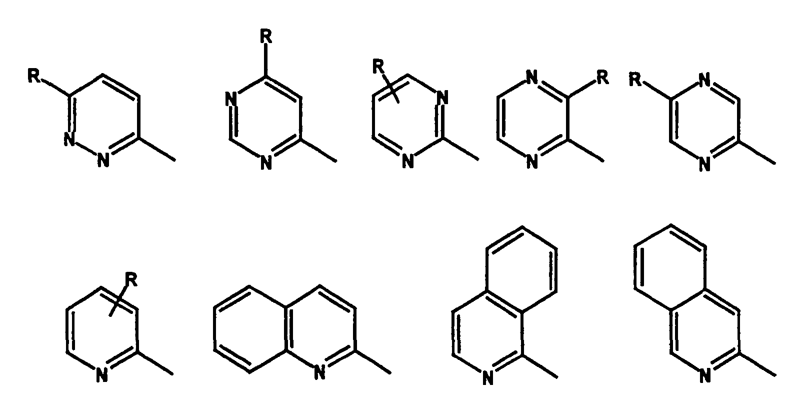 R = H or CH₃, X = NH or N-CH₃,
with the proviso that if Het = 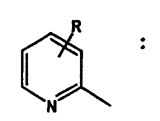 R is not H,
as well as the pharmaceutically acceptable salts thereof.

4. A compound according to claim 1, namely 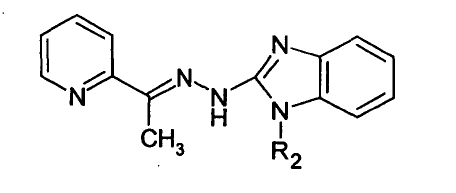 wherein R₂ = ethyl, propyl, sec.-propyl, butyl, *tert*.-butyl, allyl, cyclopropyl, phenyl, benzyl or CH₂-CH₂-O-CH₃, CH₂-CH₂-N(CH₃)₂, as well as the pharmaceutically acceptable salts thereof.

5. A compound according to claim 1, namely 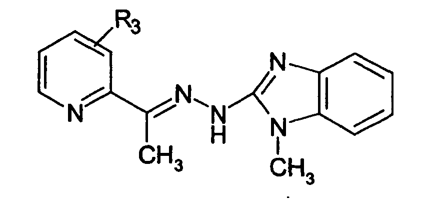 wherein R₃ is a 3, 4, 5 or 6-substituent and represents OCH₃, OH, Cl, Br, F, CH₃, CF₃, NO₂, NH₂, NHCOCH₃, N(CH₃)₂, phenyl, CN, C=NH(NH₂), C=S(NH₂), C=NH (NHOH), COOH or COOR₄, wherein R₄ is an aliphatic residue, a phenyl group or CONR₅R₆, wherein R₅ and R₆ are H, an aliphatic substituent or a phenyl group, as well as the pharmaceutically acceptable salts thereof.

6. A method for preparing a compound of the general formula 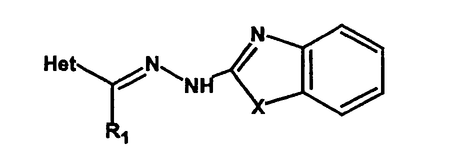 wherein Het = 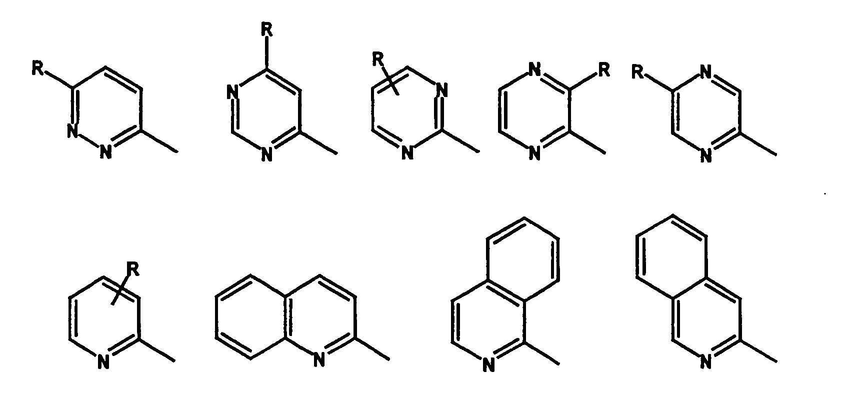 and wherein R = H, CH₃, OCH₃, OH, Cl, Br, F, CF₃, NO₂, NH₂, NHCOCH₃, N(CH₃)₂, phenyl, CN, C=NH(NH₂), C=S(NH₂), C=NH(NHOH), COOH or COOR₄, wherein R₄ = an aliphatic residue or a phenyl group, or CONR₅R₆, wherein R₅ and R₆ are H, an aliphatic substituent or a phenyl group,
R₁ = H, methyl, ethyl, propyl, *iso*-propyl, butyl, *tert*.-butyl, cyclopropyl, cyclohexyl, phenyl, benzyl, or 2-pyridyl, and X = NH or N-R₂, wherein R₂ = methyl, ethyl, propyl, *sec.*-propyl, butyl, *tert*.-butyl, allyl, cyclopropyl, phenyl, benzyl, CH₂-CH₂-O-CH₃ or CH₂-CH₂-N(CH₃)₂, **characterised in that** a ketone of the general formula (III) 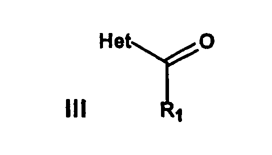 wherein Het and R₁ are as defined above, is reacted with a hydrazine of the general formula (II) 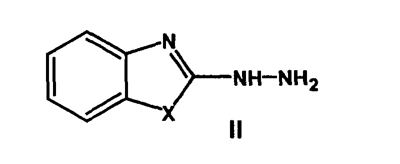 wherein X is as defined above.

7. A method according to claim 6, **characterised in that** the reaction is carried out in methanol or ethanol.

8. A method according to claim 6 or 7, **characterised in that** the reaction is carried out in the presence of a catalytic amount of a suitable acid, such as acetic acid, hydrochloric acid or sulphuric acid.

9. A compound of the general formula: 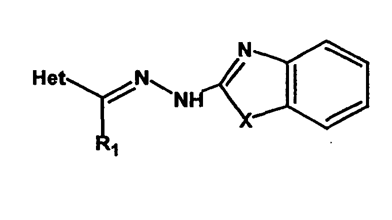 wherein Het= 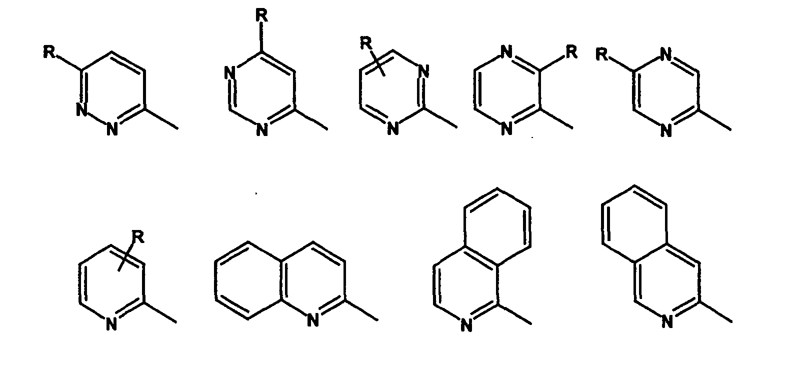 and wherein R = H, CH₃, OCH₃, OH, Cl, Br, F, CF₃, NO₂, NH₂, NH-COCH₃, N(CH₃)₂, phenyl, CN, C=NH(NH₂), C=S(NH₂), C=NH(NHOH), COOH or COOR₄, wherein R₄ = an aliphatic residue or a phenyl group, or CONR₅R₆, wherein R₅ and R₆ are H, an aliphatic substituent or a phenyl group,
R₁ is H, methyl, ethyl, propyl, *iso*-propyl, butyl, *tert.*-butyl, cyclopropyl, cyclohexyl, phenyl, benzyl, or 2-pyridyl, and X = NH or N-R₂, wherein R₂ = methyl, ethyl, propyl, *sec*.-propyl, butyl, *tert*.-butyl, allyl, cyclopropyl, phenyl, benzyl, CH₂-CH₂-O-CH₃ or CH₂-CH₂-N(CH₃)₂, as well as the pharmaceutically acceptable salts thereof, to be used as a medicament,
with the proviso that if Het= 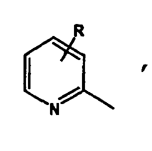 wherein R = H,
in case X = NH: R₁ is not methyl,
in case X = N-R₂ with R₂ = CH₃: R₁ is not methyl;
as well as the pharmaceutically acceptable salts thereof.

10. A compound as defined in claim 9 to be used as an anticancer therapeutic agent.

11. A compound as defined in claim 9 to be used as an anti-tumour therapeutic agent.

12. The use of a compound as defined in claim 9 in the preparation of a medicament for the treatment of cancer of the colon, breast, ovaries or uterus.

## Revendications

1. Composé de formule générale : 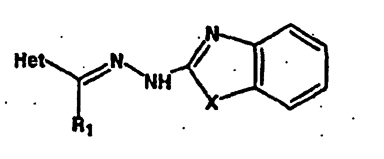 dans laquelle Het = 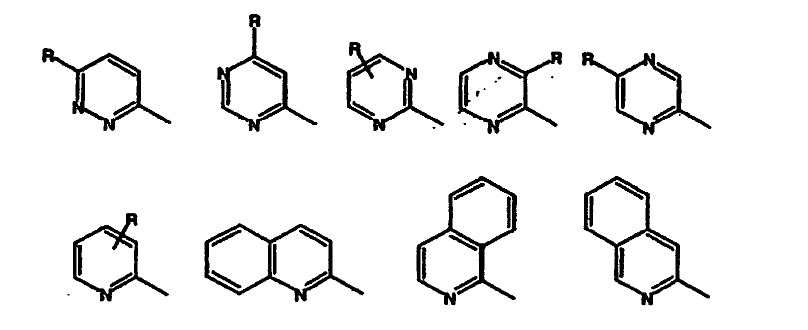 et dans laquelle R = H, CH₃, OCH₃, OH, Cl, Br, F, CF₃, NO₂, NH₂, NHCOCH₃, N(CH₃)₂, phényle, CN, C=NH(NH₂), C=S(NH₂), C=NH (NHOH), COOH ou COOR₄, où R₄ = radical aliphatique ou groupement phényle, ou CONR₅R₆, où R₅ et R₆H représentent un substituant aliphatique ou un groupement phényle,
R₁ = H, méthyle, éthyle, propyle, isopropyle, butyle, tert-butyle, cyclopropyle, cyclohexyle, phényle, benzyle ou 2-pyridyle, et
X = NH ou représente N-R₂, où R₂ = méthyle, éthyle, propyle, sec-propyle, butyle, tert-butyle, allyle, cyclopropyle, phényle, benzyle, CH₂-CH₂-O-CH₃ ou CH₂-CH₂-N(CH₃)₂,
avec comme condition que, lorsque Het = 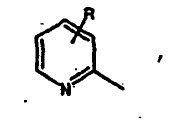 où R = H,
dans le cas où X = NH : R₁ ne représente pas un méthyle,
dans le cas où X = N-R₂ avec R₂ = CH₃ : R₁ ne représente pas un méthyle;
ainsi que ses sels pharmaceutiquement acceptables.

2. Composé selon la revendication 1, ayant la formule : 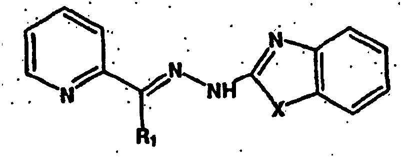 dans laquelle R₁ = H, méthyle, éthyle, propyle, isopropyle, butyle, tert-butyle, cyclopropyle, cyclohexyle, phényle, benzyle ou 2-pyridyle et X = NH ou NCH₃,
avec comme condition que,
lorsque X = NH : R₁ ne représente pas un méthyle et
lorsque X = NCH₃ : R₁ ne représente pas un méthyle,
ainsi que ses sels pharmaceutiquement acceptables.

3. Composé selon la revendication 1, ayant la formule : 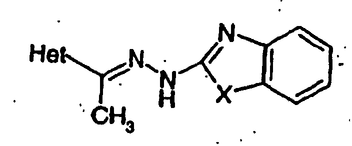 dans laquelle Het = 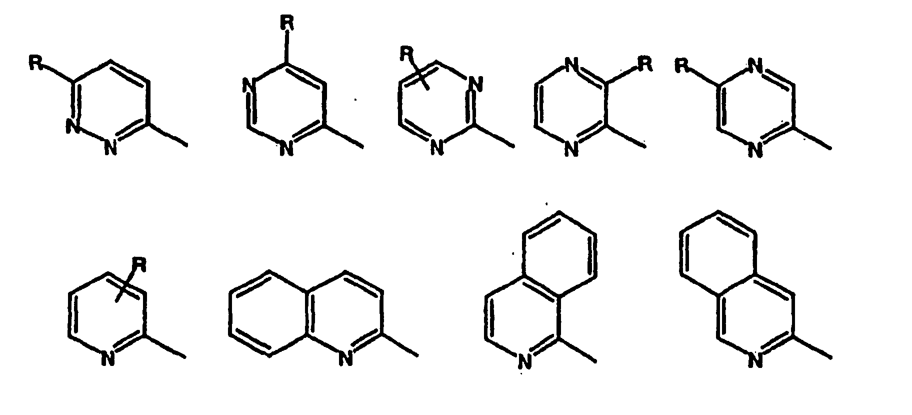 R = H ou CH₃, X = NH ou N-CH₃,
avec comme condition que, lorsque Het = 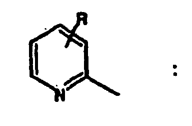 R ne représente pas H,
ainsi que ses sels pharmaceutiquement acceptables.

4. Composé selon la revendication 1, ayant la formule : 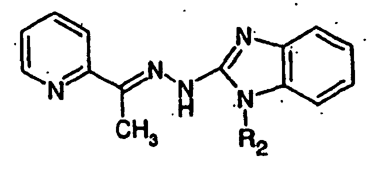 dans laquelle R₂ = éthyle, propyle, sec-propyle, butyle, tert-butyle, allyle, cyclopropyle, phényle, benzyle ou représente CH₂-CH₂-O-CH₃ ou CH₂-CH₂-N(CH₃)₂, ainsi que ses sels pharmaceutiquement acceptables.

5. Composé selon la revendication 1, ayant la formule : 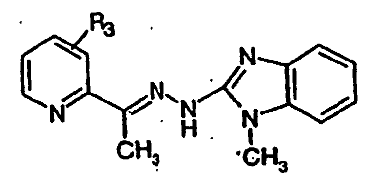 dans laquelle R₃ est un substituant en position 3, 4, 5 ou 6 et représente OCH₃, OH, Cl, Br, F, CH₃, CF₃, NO₂, NH₂, NHCOCH₃, N(CH₃)₂, phényle, CN, C=NH(NH₂), C=S(NH₂), C=NH(NHOH), COOH ou COOR₄, où R₄ représente un radical aliphatique, un groupement phényle ou CONR₅R₆, où R₅ et R₆H représentent un substituant aliphatique ou un groupement phényle, ainsi que ses sels pharmaceutiquement acceptables.

6. Procédé pour la fabrication d'un composé de formule générale : 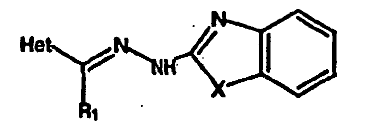 dans laquelle Het = 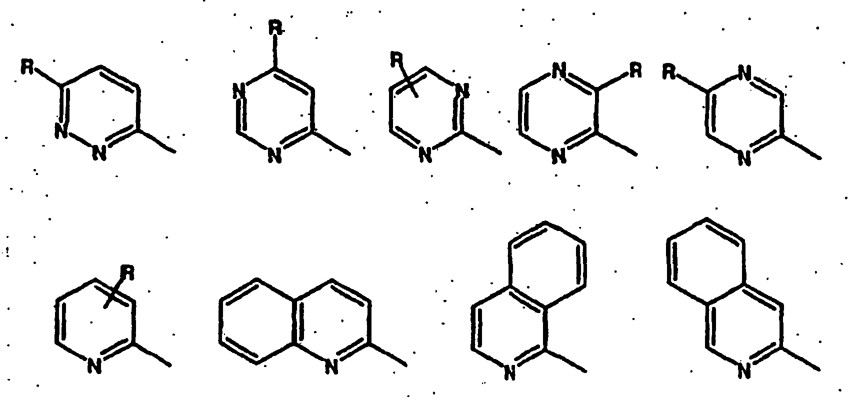 et dans laquelle R = H, CH₃, OCH₃, OH, Cl, Br, F, CF₃, NO₂, NH₂, NHCOCH₃, N(CH₃)₂, phényle, CN, C=NH(NH₂), C=S(NH₂), C=NH(NHOH), COOH ou COOR₄, où R₄ = radical aliphatique ou groupement phényle, ou CONR₅R₆, où R₅ et R₆H représentent un substituant aliphatique ou un groupement phényle,
R₁ = H, méthyle, éthyle, propyle, isopropyle, butyle, tert-butyle, cyclopropyle, cyclohexyle, phényle, benzyle ou 2-pyridyle et X = NH ou représente N-R₂, où R₂ = méthyle, éthyle, propyle, sec-propyle, butyle, tert-butyle, allyle, cyclopropyle, phényle, benzyle ou représente CH₂-CH₂-O-CH₃ ou CH₂-CH₂-N(CH₃)₂, **caractérisé en ce que** l'on fait réagir une cétone de formule générale (III) : 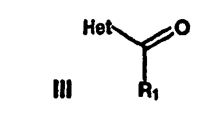 dans laquelle Het et R₁ ont les significations précités avec une hydrazine de formule générale (II) : 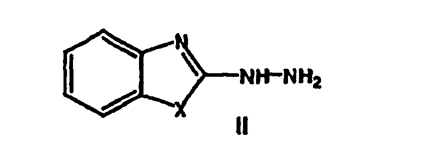 dans laquelle X a la signification mentionnée ci-dessus.

7. Procédé selon la revendication 6, **caractérisé en ce que** l'on effectue la réaction dans du méthanol ou de l'éthanol.

8. Procédé selon la revendication 6 ou 7, **caractérisé en ce que** la réaction est effectuée en présence d'une quantité catalytique d'un acide approprié, tel que l'acide acétique, l'acide chlorhydrique ou l'acide sulfurique.

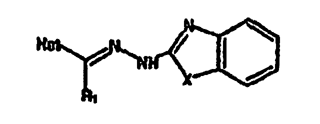

9. Composé de formule générale :
dans laquelle Het = 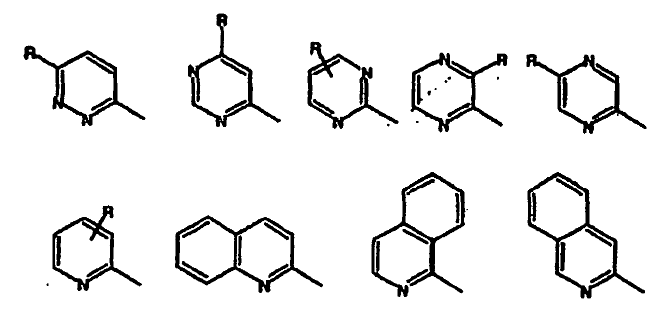 et dans laquelle R = H, CH₃, OCH₃, OH, Cl, Br, F, CF₃, NO₂, NH₂, NHCOCH₃, N(CH₃)₂, phényle, CN, C=NH(NH₂), C=S(NH₂), C=NH(NHOH), COOH ou COOR₄, où R₄ = radical aliphatique ou groupement phényle, ou CONR₅R₆, où R₅ et R₆H représentent un substituant aliphatique ou un groupement phényle,
R₁ = H, méthyle, éthyle, propyle, isopropyle, butyle, tert-butyle, cyclopropyle, cyclohexyle, phényle, benzyle ou 2-pyridyle, et
X = NH ou représente N-R₂, où R₂ = méthyle, éthyle, propyle, sec-propyle, butyle, tert-butyle, allyle, cyclopropyle, phényle, benzyle ou représente CH₂-CH₂-O-CH₃ ou CH₂-CH₂-N(CH₃)₂, ainsi que ses sels pharmaceutiquement acceptables, pour utilisation à titre de médicament.

10. Composé tel que défini dans la revendication 9,
avec comme condition que, lorsque Het = 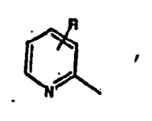 où R = H,
lorsque X = NH : R₁ ne représente pas un méthyle,
lorsque X = N-R₂ avec R₂ = CH₃ : R₁ ne représente pas un méthyle;
ainsi que ses sels pharmaceutiquement acceptables, pour une utilisation à titre de médicament anticancéreux.

11. Composé, tel que défini dans la revendication 9, pour une utilisation à titre de médicament anti-tumoral.

12. Utilisation d'un composé tel que défini dans la revendication 9, pour la fabrication d'un médicament destiné au traitement du cancer du gros intestin, du cancer du sein, du cancer des ovaires, ou du cancer de l'utérus.
